# FASCICULE DE BREVET EUROPEEN

(11) **EP 2 762 125 B1**
(45) Date de publication et mention de la délivrance du brevet: **03.08.2016**
(21) Numéro de dépôt: 14152975.0
(22) Date de dépôt: 29.01.2014
(51) Int. Cl.: A61K 8/27, A61K 8/31, A61Q 19/00, A61K 45/06, A61K 8/23, A61K 33/00, A61K 33/04, A61K 33/08, A61K 33/30, A61K 33/34, A61K 31/01, A61K 33/10, A61K 35/644

(54) **Composition dermatologique améliorée**
Verbesserte dermatologische Zusammensetzung
Improved dermatological composition

(30) Priorité: 30.01.2013 FR 1350775
(43) Date de publication de la demande: 06.08.2014
(73) Titulaire: Codexial Dermatologie, 54500 Vandeouvre les Nancy (FR)
(72) Inventeur: Treffel, Pierre, F-54500 VANDOEUVRE LES NANCY (FR)
(74) Mandataire: Rhein, Alain

(56) Documents cités:
- CA-A1- 1 036 941
- CN-A- 101 480 411
- FR-A- 838 699
- US-A- 4 388 301
- PENCE D B ET AL: "Notoedric mange in the bobcat, Felis rufus, from south Texas.", JOURNAL OF WILDLIFE DISEASES JAN 1982, vol. 18, no. 1, janvier 1982 (1982-01), pages 47-50, XP009172989, ISSN: 0090-3558

## Description

La présente invention se situe dans le domaine des produits dermatologiques.

Elle concerne plus particulièrement une composition utilisable dans des produits dermatologiques apaisants, sous forme de pommade, crème, gel, lotion, lotion-sprays, bases lavantes, ou toute autre forme galénique.

Des produits dermatologiques apaisants comprenant de l'hydroxyde de calcium et de l'huile d'olive, dosés à 50% chacun son connus.

Nous connaissons également des produits dermatologiques apaisants à 25% d'hydroxyde de calcium, 25% d'huile d'amande, 25% de graisse de laine et 25% de vaseline blanche.

Il est encore connu, dans l'état de la technique, un document de brevet FR 838 699 qui traite des perfectionnements aux émulsions et produits analogues, et qui est plus particulièrement relatif à des émulsions stables dans l'eau et comprenant de l'eau, un liquide non miscible à l'eau et de l'alumine gélatineuse activée à titre d'émulsionnant.

En outre, un certain nombre d'émulsions sous forme de produits de beauté et de toilette sont divulguées, celles-ci étant constituées d'ingrédients variés, notamment de l'huile d'amande, du saindoux benzoaté, etc. Toutefois, aucune de ces crèmes ou préparations diverses ne montre des propriétés calmantes ou apaisantes.

Le document de brevet US 4 388 301 concerne une méthode pour traiter l'acné au moyen d'une composition comportant un polysulfure soluble, et notamment de la chaux sulfurée, pour réduire la quantité de sébum produite par les glandes sébacées.

La chaux sulfurée contient des sulfures de calcium et des polysulfures, ainsi que du sulfate de calcium, des thiosulfates, des carbonates et des cendres du matériau carboné.

La composition décrite dans ce document ne consiste pas en une composition apaisante.

Le document de brevet CA 1 036 941 divulgue une composition pharmaceutique contre les irritations et les inflammations, à base notamment d'eau de chaux et d'huile végétale, comme l'huile d'olive, l'huile de carthame, l'huile de soja, l'huile de maïs, ou encore l'huile de coton.

Dans le document de brevet CN 101480411, il est décrit une composition comprenant de l'huile végétale et animale, notamment de l'huile de sésame et de l'huile de serpent, ainsi que d'autres constituants parmi lesquels de l'eau de chaux.

Néanmoins ces produits décrits dans les documents CA 1 036 941 et CN 101480411 sont susceptibles de présenter des caractéristiques très moyennes (forme galénique très grasse et risque d'oxydation de l'huile végétale), et il est souhaitable d'en améliorer les performances. De plus, leurs propriétés calmantes et apaisantes restent également à améliorer.

La présente invention a pour but de pallier au moins en partie à ces inconvénients. A cet effet elle propose une composition dermatologique comprenant de l'eau de chaux et de l'huile. Cette composition est particulière en ce qu'elle comprend en outre du sulfate de cuivre.

Grâce à ces dispositions, la composition dermatologique selon l'invention présente des propriétés apaisantes, anti-inflammatoires et neutralisantes améliorées, ainsi que des propriétés galéniques plus satisfaisantes.

Selon d'autres caractéristiques
- ladite composition peut comprendre en outre du sulfate de zinc, améliorant ainsi les propriétés antiseptiques,
- ladite composition peut comprendre en outre de l'oxyde de zinc, améliorant ainsi les propriétés apaisantes et absorbantes,
- en pourcentage massique de la composition, l'eau de chaux peut représenter entre 40% et 95%, l'huile entre 0,5% et 40%, l'oxyde de zinc entre 0,5 et 10%, le sulfate de zinc entre 0,2 et 0,4%, et le sulfate de cuivre entre 0,1 et 0,2%. De telles fourchettes de pourcentages présentent l'avantage de proposer des formes galéniques adaptées à différents types de pathologies cutanées
- la composition selon l'invention peut comprendre en outre de la cire d'abeille, de préférence à environ 2% en masse, lui conférant une plus grande douceur, et améliorant la stabilité du produit,
- ladite huile peut être une huile minérale, réduisant ainsi la réactivité à certains produits, et améliorant l'efficacité et la stabilité de la composition,
- ladite huile peut être de la paraffine liquide réduisant ainsi la réactivité à certains sulfates, et améliorant encore l'efficacité et la stabilité de la composition,
- en pourcentage massique de la composition, l'eau de chaux peut représenter 53,3%, la paraffine liquide 37%, l'oxyde de zinc 6%, le sulfate de zinc 0,35%, et le sulfate de cuivre 0,15% ; une telle composition a donné des résultats particulièrement satisfaisants en termes d'efficacité, d'acceptabilité et de tolérance.

La présente invention concerne également une utilisation de la composition selon l'invention comme pommade, notamment pour les irritations cutanées peu inflammatoires et peu ou pas suintantes.

La présente invention concerne également une utilisation de la composition selon l'invention comme crème, gel, lotion ou lotion spray, notamment pour les irritations cutanées inflammatoires avec présence d'exsudats.

La présente invention concerne encore une utilisation de la composition selon l'invention sous forme de lingettes dermatologiques, notamment pour le nettoyage de certains types de plaies (irritations, brûlures).

La présente invention concerne enfin une utilisation de la composition selon l'invention sous forme de bandes dermatologiques, notamment pour traiter et protéger certains types de plaies (irritations brûlures).

L'avantage apporté par la présente invention réside principalement en ce qu'elle propose une composition avec une meilleure efficacité calmante, apaisante et réparatrice.

Un exemple de composition pour une pommade donnant de bons résultats est la suivante :
- Eau de chaux : 53,3%
- Paraffine liquide : 37%
- Oxyde de zinc : 6%
- Cire d'abeille : 2%
- Emulsionnant de type stéarate : 1%
- Borate de sodium : 0,2%
- Sulfate de zinc : 0,35%
- Sulfate de cuivre : 0,15%.

Une telle composition permet d'obtenir une pommade avec des propriétés anti inflammatoires, tout en conservant une galénique satisfaisante.

La cire d'abeille est facultative, et permet d'améliorer la douceur du produit.

Un exemple de composition pour une lotion donnant de bons résultats est la suivante :
- Eau de chaux : 90%
- Paraffine liquide : 7,3%
- Oxyde de zinc : 1%
- Cire d'abeille : 0%
- Emulsionnant de type stéarate : 1%
- Borate de sodium : 0,2%
- Sulfate de zinc : 0,35%
- Sulfate de cuivre : 0,15%.

Dans les deux cas, le borate de sodium peut être remplacé par tout autre produit ayant des propriétés antiseptiques et co-émulsifiantes.

Un autre exemple de pommade ou lotion dermatologique selon l'invention consiste en les mêmes compositions, en remplaçant la paraffine liquide par une huile végétale, comme l'huile d'olive par exemple.

Toutefois, l'avantage particulier de la paraffine liquide réside en sa neutralité et son absence de réactivité avec entre autres les sulfates, ce qui confère à la pommade ou lotion obtenue avec de la paraffine liquide une meilleure efficacité et stabilité.

Une étude clinique concernant la pommade dermatologique selon l'invention a été réalisée.

Vingt patients ont été inclus selon les modalités du protocole, sans aucun perdus de vue. Sur les vingt cas de l'étude, dix concernent l'épilation laser effectuée par laser alexandrite 755 nm et Nd :Yag 1064 nm, six cas ont bénéficié d'un traitement de détatouage laser par laser Q-Switched 532nm et 1064 nm, et 4 patients ont été traités par lasers fractionnés ablatif (CO2 à 10600nm) et non ablatif (Er:Yag 1550 nm) pour les indications de la prise en charge du vieillissement cutané ou de correction de cicatrices. Pour ces deux dernières indications, les suites opératoires attendues sont constituées d'érythèmes et d'oedèmes, suivies de croutes, rarement de suintement, pendant une durée de 3 à 10 jours, alors que l'épilation laser est suivie classiquement d'érythème et d'oedème uniquement pendant 48 à 72 heures en moyenne.

L'évaluation des suites opératoires dans le cadre de l'étude clinique de la pommade dermatologique selon l'invention correspond parfaitement aux définitions des suites attendues de ces soins laser, avec un « profil » de l'état clinique postopératoire immédiat plus sévère pour les patients traités par laser fractionné et Q-Switched.

L'efficacité de la pommade dermatologique selon l'invention s'est avérée excellente avec un score supérieur à 7 (échelle de score de 0 à 10) pour 80 % des patients :
La restitution de l'état cutané normal à 48 h est effective pour tous les patients ayant été traités par épilation laser.

Celle-ci est encore plus significative pour les patients ayant bénéficié des autres techniques avec un retour à l'état cutané normal au 4ème jour pour 100% des patients traités dans le cadre de l'étude.

L'appréciation par les patients eux-mêmes, du bénéfice de la pommade dermatologique selon l'invention est confirmée car 75% d'entre eux trouvent la pommade dermatologique selon l'invention plus efficace et performante que les produits de l'état de la technique.

La tolérance du produit est excellente avec 95% d'absence totale de réaction, le seul cas (1/20) ayant manifesté un effet secondaire s'étant traduit par une sensation de brûlure à l'application, qui n'a pas empêché la poursuite des soins avec le produit.

Enfin, les qualités cosmétiques de la pommade dermatologique selon l'invention sont estimées extrêmement satisfaisantes pour 75% des patients (score 7/10).

La facilité d'application et l'absorption rapide du produit conviennent particulièrement aux suites de l'épilation laser.

Cette caractéristique nécessite de multiplier les applications pour une bonne efficacité dans les suites des lasers Q-switched ou fractionnés sans modification de la satisfaction d'utilisation de la pommade dermatologique selon l'invention. En outre une telle composition avec de la paraffine liquide comme huile a été testée dans des radiodermites cutanées suite à des séances de rayons utilisés dans certains traitements contre les cancers. Les effets observés ont été meilleurs qu'avec des huiles végétales.

Il en est de même dans le traitement de l'eczéma atopique.

La pommade dermatologique selon l'invention peut être préparée de la manière suivante :
Etape 1 : préparation de la phase grasse
   - ajouter l'huile de paraffine, le glycol stéarate et de la cire blanche, et chauffer le tout à 70°C,
   - ajouter l'oxyde de zinc, et agiter jusqu'à dissolution complète (température: 70°C).
Etape 2 : Préparation de la phase aqueuse
   - chauffer l'eau de chaux à 70°C,
   - ajouter le borate de sodium, et agiter jusqu'à dissolution complète (température: 70°C),
   - ajouter le sulfate de zinc, et agiter jusqu'à dissolution complète (température: 70°C),
   - ajouter le sulfate de cuivre, et agiter jusqu'à dissolution complète (température: 70°C),
Etape 3 : Formation de l'émulsion
   - introduire la phase grasse dans la phase aqueuse, et agiter jusqu'à refroidissement 30°C et vérifier l'absence de grains.

La pommade dermatologique selon l'invention peut être utilisée sur des lésions d'irritation majoritairement sèches nécessitant un environnement humide : au sein d'un produit gras type émulsion eau dans huile, des propriétés apaisantes ou anti-inflammatoires et réparatrices/protectrices ont été observées, en particulier avec la forme grasse et couvrante du produit.

L'invention peut aussi être utilisée sur des lésions avec exsudats : on utilise alors de préférence une émulsion non grasse non couvrante (crème), type huile dans eau, qui présente en plus des propriétés apaisantes ou anti-inflammatoires une action absorbante.

L'invention peut encore être utilisée sur des lésions avec exsudats importants : des formes lotion ou gel sont alors utilisées, qui présentent en plus des propriétés anti-inflammatoires une action asséchante.

La composition dermatologique selon l'invention a des propriétés apaisantes / anti-inflammatoires / neutralisantes, en particulier grâce au pH basique de l'eau de chaux. Ces propriétés peuvent être modulées en fonction de la forme galénique utilisée et donc adaptées à différentes type de lésions, d'irritations et de brulures.

Bien que l'invention ait été décrite selon un mode de réalisation particulier, elle n'y est nullement limitée, et des variantes peuvent y être apportées, ainsi que des combinaisons des variantes décrites, sans pour autant sortir du cadre de la présente invention.

## Revendications

1. Composition dermatologique comprenant de l'eau de chaux et de l'huile, **caractérisée en ce qu'**elle comprend en outre du sulfate de cuivre.

2. Composition selon la revendication précédente, comprenant en outre du sulfate de zinc.

3. Composition selon l'une des revendications précédentes, comprenant en outre de l'oxyde de zinc.

4. Composition selon la revendication précédente, dans laquelle, en pourcentage massique de la composition, l'eau de chaux représente entre 40% et 95%, l'huile entre 0,5% et 40%, l'oxyde de zinc entre 0,5 et 10%, le sulfate de zinc entre 0,2 et 0,4%, et le sulfate de cuivre entre 0,1 et 0,2%.

5. Composition selon l'une des revendications précédentes, comprenant en outre de la cire d'abeille, de préférence à environ 2% en masse.

6. Composition selon l'une des revendications précédentes, dans laquelle ladite huile est une huile minérale, en particulier de la paraffine liquide.

7. Composition selon la revendication précédente, dans laquelle, en pourcentage massique de la composition, l'eau de chaux représente 53,3%, la paraffine liquide 37%, l'oxyde de zinc 6%, le sulfate de zinc 0,35%, et le sulfate de cuivre 0,15%.

8. Pommade avec une composition selon l'une des revendications précédentes, pour son utilisation en dermatologie, en particulier pour des irritations cutanées peu inflammatoires et peu ou pas suintantes.

9. Crème, gel, lotion ou lotion spray, avec une composition selon l'une des revendications 1 à 7, pour son utilisation en dermatologie, en particulier pour des irritations cutanées inflammatoires avec présence d'exsudats.

10. Lingettes ou bandes avec une composition selon l'une des revendications 1 à 7, pour son utilisation en dermatologie, en particulier pour le nettoyage, le soin ou la protection de certains types de plaies, par exemple des irritations ou des brûlures.

## Patentansprüche

1. Dermatologische Zusammensetzung, umfassend Kalkwasser und Öl, **dadurch gekennzeichnet, dass** sie außerdem Kupfersulfat umfasst.

2. Zusammensetzung nach dem vorhergehenden Anspruch, umfassend außerdem Zinksulfat.

3. Zusammensetzung nach einem der vorhergehenden Ansprüche, umfassend außerdem Zinkoxid.

4. Zusammensetzung nach dem vorhergehenden Anspruch, bei der das Kalkwasser in Gewichtsprozent der Zusammensetzung zwischen 40% und 95%, das Öl zwischen 0,5% und 40%, das Zinkoxid zwischen 0,5 und 10%, das Zinksulfat zwischen 0,2 und 0,4% und das Kupfersulfat zwischen 0,1 und 0,2% darstellt.

5. Zusammensetzung nach einem der vorhergehenden Ansprüche, umfassend außerdem Bienenwachs, vorzugsweise etwa 2 Gewichtsprozent.

6. Zusammensetzung nach einem der vorhergehenden Ansprüche, bei der das Öl ein Mineralöl, insbesondere flüssiges Paraffin, ist.

7. Zusammensetzung nach dem vorhergehenden Anspruch, bei der das Kalkwasser in Gewichtsprozent der Zusammensetzung 53,3%, das flüssiges Paraffin 37%, das Zinkoxid 6%, das Zinksulfat 0,35 % und das Kupfersulfat 0,15% darstellt.

8. Salbe mit einer Zusammensetzung nach einem der vorhergehenden Ansprüche, zur Verwendung in der Dermatologie, insbesondere für wenig entzündliche und wenig oder gar nicht nässende Hautreizungen.

9. Creme, Gel, Lotion oder Spraylotion mit einer Zusammensetzung nach einem der Ansprüche 1 bis 7, zur Verwendung in der Dermatologie, insbesondere für entzündliche Hautreizungen mit Anwesenheit von Exsudaten.

10. Wischtücher oder Bänder mit einer Zusammensetzung nach einem der Ansprüche 1 bis 7, zur Verwendung in der Dermatologie, insbesondere zur Reinigung, Pflege oder zum Schutz von gewissen Arten von Wunden, beispielsweise von Reizungen oder Brandwunden.

## Claims

1. A dermatological composition comprising limewater and oil, wherein it comprises in addition copper sulfate.

2. The composition according to the preceding claim, comprising in addition zinc sulfate.

3. The composition according to one of the preceding claims, comprising in addition zinc oxide.

4. The composition according to the preceding claim, wherein the limewater represents, in percentage by weight of the composition, between 40% and 95%, the oil between 0.5% and 40%, the zinc oxide between 0,5 and 10%, the zinc sulfate between 0.2 and 0.4%, and the copper sulfate between 0.1 and 0.2%.

5. The composition according to one of the preceding claims, comprising in addition beeswax, preferably at about 2% by weight.

6. The composition according to one of the preceding claims, wherein said oil is a mineral oil, in particular liquid paraffin.

7. The composition according to the preceding claim, wherein the limewater represents, in percentage by weight of the composition, 53.3%, the liquid paraffin 37%, the zinc oxide 6%, the zinc sulfate 0.35 %, and the copper sulfate 0.15%.

8. An ointment with a composition according to one of the preceding claims, for use in dermatology, in particular for mildly inflammatory and mildly or non-oozing skin irritation.

9. A cream, gel, lotion or spray lotion, with a composition according to one of claims 1 to 7, for use in dermatology, in particular for inflammatory skin irritation with presence of exudates.

10. Wipes or sheets with a composition according to one of claims 1 to 7, for use in dermatology, in particular for cleaning, caring or protecting some types of wounds, for example irritations or burns.
